# EUROPEAN PATENT SPECIFICATION

(11) **EP 1 472 259 B1**
(45) Date of publication and mention of the grant of the patent: **02.09.2009**
(21) Application number: 03734703.6
(22) Date of filing: 28.01.2003
(51) Int. Cl.: C07D 487/22, A61K 31/409, A61K 49/00, A61P 35/00, C07D 257/00

(54) **TETRAPYRROLIC MACROCYCLES AS PHOTODYNAMIC AGENTS**
TETRAPYRROL-MACROCYCLEN ALS PHOTODYNAMISCHE MITTEL
MACROCYCLES TETRAPYRROLIQUES UTILISES COMME AGENTS PHOTODYNAMIQUES

(30) Priority: 01.02.2002 PT 10272102
(43) Date of publication of application: 03.11.2004
(73) Proprietor: CIPAN-Companhia Industrial Produtora De Antibioticos, S.A., 2601-962 Castanheira do Ribatejo (PT)
(72) Inventor: ROCHA GONCALVES, Antonio, 2600-726 Castanheira do Ribatejo (PT); PINEIRO, Marta, 2 600-726 Castanheira do Ribatejo (PT); SERRA, Armenio, C., 2600-726 Castanheira do Ribatejo (PT)
(74) Representative: Polypatent
(86) International application number: PCT/EP2003/000829
(87) International publication number: WO 2003/064427

(56) References cited:
- EP-A- 0 337 601
- WO-A-00/61584
- WO-A-01/53300
- WO-A-01/66550
- WO-A-01/74398
- WO-A-01/96344
- WO-A-88/07988
- WO-A-97/00874
- CZ-B- 278 391
- DATABASE CROSSFIRE BEILSTEIN [Online] Beilstein Institut zur Förderung der Chemischen Wissenschaften, Frankfurt am Main, DE; A. WIEHE ET AL.: Database accession no. 9242238,9235110 XP002236457 & ORG. LETT., vol. 4, no. 22, 2002, pages 3807-3810,
- DATABASE CROSSFIRE BEILSTEIN [Online] Beilstein Institut zur Förderung der Chemischen Wissenschaften, Frankfurt am Main, DE; T. YAMADA ET AL.: Database accession no. 8670816,8670721 XP002236458 & CHEM. LETT., vol. 6, 2000, pages 644-645,
- DATABASE CROSSFIRE BEILSTEIN [Online] Beilstein Institut zur Förderung der Chemischen Wissenschaften, Frankfurt am Main, DE; E. BACIOCCHI ET AL.: Database accession no. 8535340 XP002236459 & EUR. J. ORG. CHEM., vol. 12, 1999, pages 3281-3286,
- DATABASE CROSSFIRE BEILSTEIN [Online] Beilstein Institut zur Förderung der Chemischen Wissenschaften, Frankfurt am Main, DE; V. KNYUKSHTO ET AL.: Database accession no. 8248071 XP002236460 & CHEM. PHYS. LETT., vol. 297, no. 1-2, 1998, pages 97-108,
- DATABASE CROSSFIRE BEILSTEIN [Online] Beilstein Institut zur Förderung der Chemischen Wissenschaften, Frankfurt am Main, DE; S. SONGCA ET AL.: Database accession no. 7849370 XP002236461 & S. AFR. J. CHEM., vol. 50, no. 1, 1997, pages 40-47,
- DATABASE CROSSFIRE BEILSTEIN [Online] Beilstein Institut zur Förderung der Chemischen Wissenschaften, Frankfurt am Main, DE; R. A. W. JOHNSTONE ET AL.: Database accession no. 7790820,7790624 XP002236462 & HETEROCYCLES, vol. 43, no. 7, 1996, pages 1423-1438,
- DATABASE CROSSFIRE BEILSTEIN [Online] Beilstein Institut zur Förderung der Chemischen Wissenschaften, Frankfurt am Main, DE; T. WIJESEKERA ET AL.: Database accession no. 7564773 XP002236463 & BULL. SOC. CHIM. FR., vol. 133, no. 7-8, 1996, pages 765-776,
- DATABASE CROSSFIRE BEILSTEIN [Online] Beilstein Institut zur Förderung der Chemischen Wissenschaften, Frankfurt am Main, DE; B. PIETZYK ET AL.: Database accession no. 7459852,7458153 XP002236464 & PHARMAZIE, vol. 50, no. 11, 1995, pages 747-750,
- DATABASE CROSSFIRE BEILSTEIN [Online] Beilstein Institut zur Förderung der Chemischen Wissenschaften, Frankfurt am Main, DE; S. RAO, V. KRISHNAN: Database accession no. 7060993 XP002236465 & J. MOL. STRUCT., vol. 327, 1994, pages 279-286,
- DATABASE CROSSFIRE BEILSTEIN [Online] Beilstein Institut zur Förderung der Chemischen Wissenschaften, Frankfurt am Main, DE; C. M. BARZILAY ET AL.: Database accession no. 7329620 XP002236466 & CHEM. EUROP. J., vol. 1, no. 4, 1995, pages 222-231,
- DATABASE CROSSFIRE BEILSTEIN [Online] Beilstein Institut zur Förderung der Chemischen Wissenschaften, Frankfurt am Main, DE; S. BANFI ET AL.: Database accession no. 6952231,6365366,4349029 XP002236467 & TETRAHEDRON, vol. 50, no. 30, 1994, pages 9025-9036,
- DATABASE CROSSFIRE BEILSTEIN [Online] Beilstein Institut zur Förderung der Chemischen Wissenschaften, Frankfurt am Main, DE; S. BANFI ET AL.: Database accession no. 6171503 XP002236468 & GAZZ. CHIM. ITAL., vol. 123, no. 7, 1993, pages 409-415,
- DATABASE CROSSFIRE BEILSTEIN [Online] Beilstein Institut zur Förderung der Chemischen Wissenschaften, Frankfurt am Main, DE; L. R. NUDY ET AL.: Database accession no. 5999052,5155771,4563191,4548949,1181631 XP002236469 & HETEROCYCLES, vol. 26, no. 7, 1987, pages 1797-1803,
- DATABASE CROSSFIRE BEILSTEIN [Online] Beilstein Institut zur Förderung der Chemischen Wissenschaften, Frankfurt am Main, DE; L.-C. GONG: Database accession no. 5695450,602416 XP002236470 & CAN. J. CHEM., vol. 63, 1985, pages 406-411,
- DATABASE CROSSFIRE BEILSTEIN [Online] Beilstein Institut zur Förderung der Chemischen Wissenschaften, Frankfurt am Main, DE; R. BONNET ET AL.: Database accession no. 5387493,5375213,5369706,600142,600141 XP002236471 & J. CHEM. SOC. FARADAY TRANS., vol. 88, no. 6, 1992, pages 763-770,
- DATABASE CROSSFIRE BEILSTEIN [Online] Beilstein Institut zur Förderung der Chemischen Wissenschaften, Frankfurt am Main, DE; Y. NARUTA ET AL.: Database accession no. 5387492,5387268 XP002236472 & TETRAHEDRON LETT., vol. 33, no. 8, 1992, pages 1069-1072,
- DATABASE CROSSFIRE BEILSTEIN [Online] Beilstein Institut zur Förderung der Chemischen Wissenschaften, Frankfurt am Main, DE; L. A. ANDREWS ET AL.: Database accession no. 5199282,5203537,5206405,5208702,5211989 XP002236473 & J. CHEM. SOC. PERKIN TRANS. 1, 1988, pages 1735-1738,
- DATABASE CROSSFIRE BEILSTEIN [Online] Beilstein Institut zur Förderung der Chemischen Wissenschaften, Frankfurt am Main, DE; P. S. CLEZY ET AL.: Database accession no. 5368038 XP002236474 & AUST. J. CHEM., vol. 45, no. 4, 1992, pages 703-712,
- DATABASE CROSSFIRE BEILSTEIN [Online] Beilstein Institut zur Förderung der Chemischen Wissenschaften, Frankfurt am Main, DE; O. V. MALKOVA ET AL.: Database accession no. 5216348,1203855 XP002236475 & J. ORG. CHEM. USSR (ENGL. TRANSL.)24, vol. 24, no. 7, 1988, pages 1393-1396, 7
- DATABASE CROSSFIRE BEILSTEIN [Online] Beilstein Institut zur Förderung der Chemischen Wissenschaften, Frankfurt am Main, DE; A. S. SEMEIKIN ET AL.: Database accession no. 4649678,4649677,4649676,4634449 XP002236476 & CHEM. HETEROCYCL. COMPD. (ENGL. TRANSL.), vol. 22, no. 6, 1986, pages 629-632,
- DATABASE CROSSFIRE BEILSTEIN [Online] Beilstein Institut zur Förderung der Chemischen Wissenschaften, Frankfurt am Main, DE; J. S. LINDSEY, R. W. WAGNER: Database accession no. 4641929,1208205 XP002236477 & J. ORG. CHEM., vol. 54, 1989, pages 828-836,
- DATABASE CROSSFIRE BEILSTEIN [Online] Beilstein Institut zur Förderung der Chemischen Wissenschaften, Frankfurt am Main, DE; O. M. MINNETIAN ET AL.: Database accession no. 4637899,4631884,4629346,4629333 XP002236478 & J. ORG. CHEM., vol. 54, 1989, pages 5567-5574,
- DATABASE CROSSFIRE BEILSTEIN [Online] Beilstein Institut zur Förderung der Chemischen Wissenschaften, Frankfurt am Main, DE; J. S. MANKA, D. S. LAWRENCE: Database accession no. 4611511 XP002236479 & TETRAHEDRON. LETT., vol. 30, no. 50, 1989, pages 6989-6992,
- DATABASE CROSSFIRE BEILSTEIN [Online] Beilstein Institut zur Förderung der Chemischen Wissenschaften, Frankfurt am Main, DE; FISCHER, NEUMANN: Database accession no. 378981,380872 XP002236480 & JUSTUS LIEBIGS ANN. CHEM., vol. 494, 1932, page 225
- DATABASE CROSSFIRE BEILSTEIN [Online] Beilstein Institut zur Förderung der Chemischen Wissenschaften, Frankfurt am Main, DE; FISCHER, ROESE: Database accession no. 382553 XP002236481 & CHEM. BER., vol. 46, 1913, page 2460
- DATABASE CROSSFIRE BEILSTEIN [Online] Beilstein Institut zur Förderung der Chemischen Wissenschaften, Frankfurt am Main, DE; BONNETT ET AL.: Database accession no. 601054,1199467,1235572 XP002236482 & J. CHEM. SOC. C, 1966, page 1600
- DATABASE CROSSFIRE BEILSTEIN [Online] Beilstein Institut zur Förderung der Chemischen Wissenschaften, Frankfurt am Main, DE; R. L. NUDY ET AL.: Database accession no. 4584455 XP002236483 & J. HETEROCYCL. CHEM., vol. 19, 1982, pages 1589-1590,
- DATABASE CROSSFIRE BEILSTEIN [Online] Beilstein Institut zur Förderung der Chemischen Wissenschaften, Frankfurt am Main, DE; A. M. D'A. R. GONSALVES ET AL.: Database accession no. 4348244,4348243 XP002236484 & TETRAHEDRON LETT., vol. 32, no. 10, 1991, pages 1355-1358,
- DATABASE CROSSFIRE BEILSTEIN [Online] Beilstein Institut zur Förderung der Chemischen Wissenschaften, Frankfurt am Main, DE; R. BONNETT ET AL.: Database accession no. 4283575,4283574 XP002236485 & J. CHEM. RES. MINIPRINT, vol. 5, 1990, pages 1015-1043,
- DATABASE CROSSFIRE BEILSTEIN [Online] Beilstein Institut zur Förderung der Chemischen Wissenschaften, Frankfurt am Main, DE; JOHNSON, OLDFIELD: Database accession no. 4053498 XP002236486 & J. CHEM. SOC. C, 1966, page 794
- DATABASE CROSSFIRE BEILSTEIN [Online] Beilstein Institut zur Förderung der Chemischen Wissenschaften, Frankfurt am Main, DE; J. P. COLLMAN ET AL.: Database accession no. 3645287 XP002236487 & J. AMER. CHEM. SOC., vol. 112, no. 8, 1990, pages 2986-2998,
- DATABASE CROSSFIRE BEILSTEIN [Online] Beilstein Institut zur Förderung der Chemischen Wissenschaften, Frankfurt am Main, DE; BILLIG, BAKER: Database accession no. 1236126,1236124 XP002236488 & CHEM. IND. (LONDON), 1969, page 654
- DATABASE CROSSFIRE BEILSTEIN [Online] Beilstein Institut zur Förderung der Chemischen Wissenschaften, Frankfurt am Main, DE; SMITH ET AL.: Database accession no. 1196909 XP002236489 & J. AMER. CHEM. SOC., vol. 101, 1979, page 5953
- DATABASE CROSSFIRE BEILSTEIN [Online] Beilstein Institut zur Förderung der Chemischen Wissenschaften, Frankfurt am Main, DE; EVANS, SMITH: Database accession no. 1201984 XP002236490 & TETRAHEDRON LETT., 1977, page 3079
- DATABASE CROSSFIRE BEILSTEIN [Online] Beilstein Institut zur Förderung der Chemischen Wissenschaften, Frankfurt am Main, DE; QUIMBY, LONGO: Database accession no. 1203856 XP002236491 & J. AMER. CHEM. SOC., vol. 97, 1975, page 5111
- DATABASE CROSSFIRE BEILSTEIN [Online] Beilstein Institut zur Förderung der Chemischen Wissenschaften, Frankfurt am Main, DE; S. BANFI ET AL.: Database accession no. 1206059 XP002236492 & GAZZ. CHIM. ITAL., vol. 120, no. 7, 1990, pages 435-441,
- DATABASE CROSSFIRE BEILSTEIN [Online] Beilstein Institut zur Förderung der Chemischen Wissenschaften, Frankfurt am Main, DE; S. M. S. CHAUHAN ET AL.: Database accession no. 1208206 XP002236493 & SYNTH. COMMUN., vol. 31, no. 1, 2001, pages 33-38,
- DATABASE CA [Online] CHEMICAL ABSTRACTS SERVICE, COLUMBUS, OHIO, US; A. J. F. N. SOBRAL ET AL.: "5,15-Diaryl-.beta.-substituted-porphyrina to-manganese(III) chlorides as probes for structure-activity relationships in porphyrin-based epoxidation catalysis" Database accession no. 2002:102688 XP002236494 & J. PORPHYRINS PHTHALOCYANINES, vol. 5, no. 12, 2001, pages 861-866,
- CHEMICAL ABSTRACTS, vol. 134, no. 23, 2001 Columbus, Ohio, US; abstract no. 334118a, M. PINEIRO ET AL.: "Singlet oxygen quantum yields from halogenated chlorins: potential new photodynamic therapy agents" page 1427; XP002236443 & J. PHOTOCHEM. PHOTOBIOL. A, vol. 138, no. 2, 2001, pages 147-157,
- CHEMICAL ABSTRACTS, vol. 133, no. 9, 28 August 2000 (2000-08-28) Columbus, Ohio, US; abstract no. 120179b, S. M. S. CHAUHAN ET AL.: "Clay catalyzed synthesis of 5,10,15,20-tetrakis(2,6-dichlorophenyl)-po rphyrin and related porphyrins" page 701; XP002236444 & INDIAN J. HETEROCYCL. CHEM., vol. 9, no. 3, 2000, pages 231-232,
- CHEMICAL ABSTRACTS, vol. 126, no. 3, 20 January 1997 (1997-01-20) Columbus, Ohio, US; abstract no. 41831k, S. BANFI ET AL.: "Mn(III)-tetraarylporphyrins bearing covalently bonded crown-ethers: synthesis and catalytic activity in 1-dodecene epoxidation promoted by aqueous HOCl/OCl-" page 1444; XP002236445 & J. MOL. CATAL. A: CHEM, vol. 113, no. 1-2, 1996, pages 369-377,
- CHEMICAL ABSTRACTS, vol. 119, no. 4, 26 July 1993 (1993-07-26) Columbus, Ohio, US; abstract no. 37307g, T. P. G. SUTTER ET AL.: "Steric and inductive effects on the basicity of porphyrins and on the site of protonation of porphyrin dianions: radiolytic reduction of porphyrins and metalloporphyrins to chlorins or phlorins" page 670; XP002236446 & J. CHEM. SOC., FARADAY TRANS., vol. 89, no. 3, 1993, pages 495-502,
- CHEMICAL ABSTRACTS, vol. 115, no. 12, 23 September 1991 (1991-09-23) Columbus, Ohio, US; abstract no. 122268g, H. C. TUNG ET AL.: "Electron-transfer thermodynamics, valence-electron hybridization, and bonding of the meso-tetrakis(2,6-dichlorophenyl)porphinat o complexes of manganese, iron, cobalt, nickel, copper, silver, and zinc of the P+Mn(O) and .P+Fe)O) oxene adducts" page 516; XP002236447 & LANGMUIR, vol. 7, no. 8, 1991, pages 1635-1641,
- CHEMICAL ABSTRACTS, vol. 111, no. 14, 2 October 1989 (1989-10-02) Columbus, Ohio, US; abstract no. 122655y, A. S. RICHERT ET AL.: "Ligand-centered redox processes for manganese, iron and cobalt, MnL3, FeL3, and CoL3, complexes (L = acetylacetonate, 8-quinolinate, picolinate, 2,2'-bipyridyl, 1,10-phenanthroline) and for their tetrakis(2,6-dichlorophenyl)porphinato complexes[M(por)]" page 497; XP002236448 & INORG. CHEM., vol. 28, no. 12, 1989, pages 2471-2475,
- CHEMICAL ABSTRACTS, vol. 90, no. 24, 11 June 1979 (1979-06-11) Columbus, Ohio, US; abstract no. 195192c, YU. V. GLAZKOV ET AL.: "EPR spectra of cation radicals of halogen derivatives of porphyrins" page 563; XP002236449 & ZH. PRIKL. SPEKTROSK., vol. 30, no. 3, 1979, pages 491-496,
- CHEMICAL ABSTRACTS, vol. 114, no. 2, 14 January 1991 (1991-01-14) Columbus, Ohio, US; abstract no. 16589g, Y. WATANABE ET AL.: "Transformation of Fe(III)TMP N-oxide to a two-electron oxidized equivalent of Fe(III)TMP complex (TMP = 5,10,15,20-tetramesitylporphyrin)" page 690; XP002236450 & J. CHEM. SOC., CHEM. COMMUN., no. 18, 1990, pages 1262-1264,
- DATABASE CA [Online] CHEMICAL ABSTRACTS SERVICE, COLUMBUS, OHIO, US; S. P. SONGCA: "In-vitro activity and tissue distribution of new fluorinated meso-tetrahydroxyphenylporphyrin photosensitizers" Database accession no. 2001:920301 XP002236495 & J. PHARM. PHARMACOL., vol. 53, no. 11, 2001, pages 1469-1476,
- CHEMICAL ABSTRACTS, vol. 134, no. 23, 2001 Columbus, Ohio, US; abstract no. 334118a, M. PINEIRO ET AL.: "Singlet oxygen quantum yields from halogenated chlorins: potential new photodynamic therapy agents" page 1427; XP002236451 & J. PHOTOCHEM. PHOTOBIOL., vol. 138, no. 2, 2001, pages 147-157,
- CHEMICAL ABSTRACTS, vol. 134, no. 16, 2001 Columbus, Ohio, US; abstract no. 222548j, S. P. SONGCA, B. MBATHA: "Synthesis of solubilized meso-tetrahydroxyphenylporphyrin photosensitizers by substitution with 2,3-dihydroxy-1-propyloxy groups" page 736; XP002236452 & S. AFR. J. CHEM., vol. 53, no. 2, 2000, pages 113-118,
- CHEMICAL ABSTRACTS, vol. 134, no. 13, 2001 Columbus, Ohio, US; abstract no. 183383e, S. P. SONGCA, B. MBATHA: "Solubilization of meso-tetrahydroxyphenylporphyrin photosensitizers by substitution with fluorine and with 2,3-dihydroxy-1-propyloxy groups" page 1155; XP002236453 & J. PHARM. PHARMACOL., vol. 52, no. 11, 2000, pages 1361-1367,
- CHEMICAL ABSTRACTS, vol. 130, no. 6, 8 February 1999 (1999-02-08) Columbus, Ohio, US; abstract no. 73734e, M. PINEIRO ET AL.: "Photoacoustic measuremets of porphyrin triplet-state quantum yields and singlet-oxygen efficiencies" page 1384; XP002236454 & CHEM. -- EUR. J., vol. 4, no. 11, 1998, pages 2299-2307,

## Description

This invention concerns new macrocyclic pyrrolic compounds with appropriate amphiphilicity and halogen substituents with the objective of optimising photophysical, photochemical and pharmacological properties of these compounds so that they may be used in photodynamic therapy (PDT) and photodiagnosis . The present invention also includes new synthetic processes for the preparation of this particular type of compounds.

The included structures are: 5,10,15,20-tetrakisphenylporphyrins, 5,10,15,20-tetrakisphenylchlorins, and 5,10,15,20-tetrakisphenylbacteriochlorins with a hydroxyl group in the *meta* position and bromine in the *ortho* and/or *para* positions of the phenyl rings. The substituents of positions 2,3,7,8,12,13,17 and 18 may be hydrogen, halogen atoms or alkyl, vinyl, carboxyl, carboxylalkyl, alkoxycarbonyl, alkoxycarbonylalkyl, alkoxycarbonylaryl, acetoxy groups or hydroxyalkylgroups. Also included are the analogous porphyrins, chlorins and bacteriochlorins that have halogens or hydrogens, instead of phenyl groups, directly bonded to one or more of the 5,10,15 and 20 positions of the macrocycle.

The invention also comprises the use of these compounds in photodynamic therapy and photodiagnosis.

### Background of the invention

Photodynamic therapy is presently a well established technique which allows for the treatment of some oncological and non-oncological diseases, by irradiation of the affected areas with visible light after a sensitizer that has been administered localizes itself preferentially in the target cells. The sensitizer is a molecule with the capacity to absorb energy from the radiation and transfer it to generate active species responsible for cellular death or necrosis that contributes to the cure process. The sensitizer is therefore a species without pharmacological activity and its function consists in the production, from inactive species present in the medium, of other species with real activity. Normally, the referred species is oxygen which is converted from its natural triplet state into the reactive singlet state. Thus, the sensitizer does not have the function of a common pharmaceutical compound but, through the interaction with light, basically generates the species that will carry out the pharmacological function. In order to carry out this role as a suitable sensitizer for PDT, some specific properties must be present, such as:
1. be easily transported by the blood and be selectively retained by target cellular structures;
2. absorb light efficiently from the visible region designated as the therapeutic window (600-800 nm), preferentially at a high wavelength, and transfer the energy to generate singlet oxygen with high quantum yield.
3. be easily eliminated by the organism in an adequate short time to avoid side effects.

Review papers recently published present the state of the art of the chemical, physico-chemical, biological and therapeutical studies with respect to the use of tetrapyrrolic macrocycles in PDT¹⁻⁹.

From the intense efforts that were dedicated to the preparation of compounds for use in PDT, only a small number proved to have the suitable characteristics required in order to be accepted for initiating therapeutic studies and commercialisation, table 1. None of these compounds however, fulfil all of the required characteristics for this type of application. In spite of the fact that some of the compounds that are in the final phase of development and commercialisation present high absorption coefficients in the therapeutic window, they show low quantum yields for the formation of singlet oxygen, a problem that impairs its efficacy. This fact and the diversity of target tissues imply the need for the development and search for new compounds.

**Table 1. Sensitizers on therapeutic studies and commercialization phase.**

| **Commercial name** | **λₘₐₓ(nm)** | **εₘₐₓ** | **Φ_{F}** | **Φ_{T}** | **Φ_{△}** |
|---|---|---|---|---|---|
| Photofrin® | 630 | 3000 | 0.02- 0.03 | | 0.9-0.1 |
| Npe₆ | 660 | 40000 | | | 0.8 |
| Visudyne® | 686 | 34000 | | | 0.7 |
| Foscan® | 650 | 29600 | 0.1 | 0.9 | 0.4 |
| Zn-phtalocyanine | 660 | | | | |
| Hypericine | | | | | |
| Hypocrellines | 630-700 | | | | |
| ATX-S10 | 672 | | | | |
| SQN-400 | 740 | | | | |
| Al-phtalocyanine disulphonated | 685 | | | | |
| Si-naphtalocyanine | 776 | | | | |
| Bacteriopurpurines | 800-820 | | | | |
| Purlytin® | 659 | 30300 | | 0.7 | 0.6 |
| Photochlor | 665 | 47500 | | | 0.5 |
| BOPP | 630 | | | | 0.6 |
| Antrin®, Lutrin®, Optrin® | 732 | 42000 | | | 0.6 |

The potential for the application of the tetrapyrrolic macrocycles in photodynamic therapy and complementary therapies comprises several situations:
Non-oncologic applications include vascular applications of photochemotherapy: an example is the ophthalmologic problem of age related macular degeneration (AMD) which requires sensitizers with absorption values at wavelengths higher than 700 nm; another example is the use of PDT for the prevention of restenoosis.
   Relatively to oncological applications the use of PDT in the destruction of large tumours appears to have unsatisfactory results except in a few cases of brain tumours in which the existence of a blood-brain barrier favours the concentration ratio of the sensitizer in the tumour tissue relatively to the healthy tissues. In this case light diffusion through brain tissue seems to be better than through others. Particularly important is the case of glyoblastoms, an "orphelin" pathology that has a very short survival prognosis, about one year, or less.
As examples of destruction of tumours by PDT with small area and thickness are the mouth leucodisplasies, very common in developing countries, which can be promptly and easily treated by PDT contrary to the present situation where treatment is not made for being too expensive; pre-cancerous lesion as Barrett mucosa, with increasing cases in industrialized countries, can be cured by PDT with minimal invasive treatments; actinic keratosis, with a growing number of cases due to aging of the population and increasing solar exposition, showed complete cure by PDT therapy.

The photochemistry studies that support this invention allowed for the modulation of the macrocycle structures with the purpose of getting the maximum singlet oxygen quantum yield or the maximum fluorescence quantum yield. This double feature of the invention allows for the synthesis of efficient sensitizers or alternatively good photodiagnostic compounds. To perform this latter role the molecules must show a strong tumour tropism and a very short lifetime in the body.

### Disclosure of the invention

This invention is based in the modulation of the structure of the sensitizer relatively to its photophysical and pharmacological properties. The type of substituents that have been incorporated into the macrocycles was selected with the aim of maximizing the required properties for PDT application. The selection of the adequate pattern of substitution is sustained by our own knowledge and studies in organic synthesis, photochemistry and photophysical properties as well as by the knowledge of the structure of natural tetrapyrrolic macrocycles, their physical-chemical behaviour and the specific process for the synthesis of these structures¹⁰⁻¹⁹. The synthetic methods were specifically established to allow the preparation of the required sensitizers.

As a result of our studies we concluded that a certain number of halogen atoms specifically located at the peripheric positions of the porphyrin macrocycle is a crucial factor in order to maximize the quantum yield generation of singlet oxygen by the sensitizer and is one of the essential Darts of this invention. Phenyl groups at 5, 10, 15 and 20 positions with one to three bromine atoms at *ortho* and/or *para* are particularly efficient, bromine being especially active. The presence of a hydroxyl group particularly at the *meta* positions of the mentioned phenyl groups gives the macrocycle hydrophilic properties the utility of which was already known²⁶. The capacity and specific conditions that allow bromine atoms to affect on the singlet oxygen quantum yield originating the macrocycles having the characteristics to be efficient PDT sensitizers was unknown and never described before the development of our studies. This new knowledge is the core of this invention. The type of phenyl substituents in the macrocycle can be two or more. The maximum halogen atoms, particularly bromine, directly attached to positions 5, 10, 15 and 20 or 2, 3, 7, 8, 12, 13, 17 and 18 can play an equivalent role, being also part of this invention.

The number of halogen atoms and their location on the macrocycle requires a specific tuning to ensure the maximum quantum yield of singlet oxygen. Other positions of the porphyrin macrocycle that lack halogen atoms can accommodate substituents such as alkyl, vinyl, carboxyl, carboxyalkyl, alkoxycarbonyl, alkoxycarbonylalkyl, alkoxycarbonylaryl, acetoxy or hydroxyalkyl groups in symmetrical or asymmetrical orientations in order to grant amphiphilic characteristics to the porphyrins. Groups that are present in the natural porphyrins will be chosen in order to improve the selectivity for different kinds of tumour cells and cellular components and to provide the correct lifetime in order to allow enough time for light treatment but not so long as to make elimination difficult.

With the purpose of selecting the appropriate wavelength of the radiation the compounds chosen as sensitizers can be in different oxidation states as porphyrin, chlorin or bacteriochlorin (structures I, II or III).

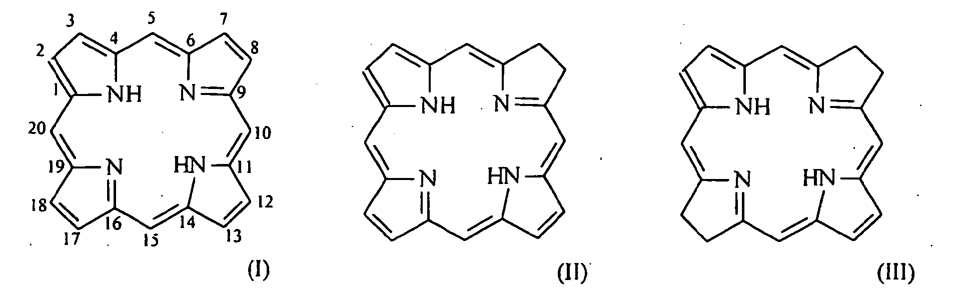

### General structures of porphyrin (I), chlorin (II) and bacteriochlorin (III)

The tetrapyrrolic macrocycles of the different types I, II or III included in this invention are identified in table 2.

**Table 2**

| Porphyrin | Substituent location | | | | | | | | | | | |
|---|---|---|---|---|---|---|---|---|---|---|---|---|
| | 2 | 3 | 7 | 8 | 12 | 13 | 17 | 18 | 5 | 10 | 15 | 20 |
| PH1 | H | H | H | H | H | H | H | H | PhOH(Z) | PhOH(Z) | PhOH(Z) | PhOH(Z) |
| PH1' | | | | | | | | | PhOH(Z) | H | PhOH(Z) | H |
| PH1'' | | | | | | | | | PhOH(Z) | X | PhOH(Z) | X |
| PH2 | X | Pr | Me | Alkyl | X | Pr | Pr | X | H | H | H | H |
| PH2' | | | | | | | | | Ph(Z) | H | Ph(Z) | H |
| PH2'' | | | | | | | | | PhOH(Z) | H | PhOH(Z) | H |
| PH3 | Me | Pr | Me | Alkyl | Me | Pr | Pr | Me | X | X | X | X |
| PH3' | | | | | | | | | Ph(Z) | H | Ph(Z) | H |
| PH3'' | | | | | | | | | Ph(Z) | X | Ph(Z) | X |
| PH3''' | | | | | | | | | PhOH(Z) | H | PhOH(Z) | H |
| PH3''' | | | | | | | | | PhOH(Z) | X | PhOH(Z) | X |
| PH4 | Me | Alkyl | Me | Alkyl | Me | Pr | Pr | Me | X | X | X | X |
| PH4' | | | | | | | | | Ph(Z) | H | Ph(Z) | H |
| PH4'' | | | | | | | | | Ph(Z) | X | Ph(Z) | X |
| PH4''' | | | | | | | | | PhOH(Z) | H | PhOH(Z) | H |
| PH4'''' | | | | | | | | | PhOH(Z) | X | PhOH(Z) | X |
| PH5 | Me | Alkyl | Alkyl | Me | Me | Pr | Pr | Me | X | X | X | X |
| PH5' | | | | | | | | | Ph(Z) | H | Ph(Z) | H |
| PH5'' | | | | | | | | | Ph(Z) | X | Ph(Z) | X |
| PH5''' | | | | | | | | | PhOH(Z) | H | PhOH(Z) | H |
| PH5'''' | | | | | | | | | PhOH(Z) | X | PhOH(Z) | X |
| PH6 | X | Alkyl | X | Alkyl | Me | Pr | Pr | Me | H | H | H | H |
| PH6' | | | | | | | | | Ph(Z) | H | Ph(Z) | H |
| PH6'' | | | | | | | | | PhOH(Z) | H | PhOH(Z) | H |
| PH6''' | | | | | | | | | PhOH(Z) | X | PhOH(Z) | X |
| PH7 | X | Alkyl | Alkyl | X | Me | Pr | Pr | Me | H | H | H | H |
| PH7' | | | | | | | | | Ph(Z) | H | Ph(Z) | H |
| PH7'' | | | | | | | | | PhOH(Z) | H | PhOH(Z) | H |
| PH7''' | | | | | | | | | PhOH(Z) | X | PhOH(Z) | X |

| | | | | | | | | | | | | |
|---|---|---|---|---|---|---|---|---|---|---|---|---|
| X = F, Cl, Br, I Ph(Z) = 2-mono-substituted phenyl, 2,4 di-substituted or 2,4,6- tri-substituted; Z = Br, H PhOH(Z)=3-hydroxyphenyl-2-mono-substituted, 2,4-di-substituted or 2,4,6- tri-substituted;Z = H, Br, Pr = -CHAlkyl = -(CH₂)ₙ-CH₃, n = 1, 2...14 | | | | | | | | | | | | |

Synthetic processes that were developed are also considered as part of this invention.

The halogenated hydroxyaldehydes required for the synthesis of the 5,10,15,20 tetrakisarylmacrocycles are prepared according to described methods ^{20.21}.

5,10,15,20-Tetrakisarylporphyrins are prepared from pyrrol and the appropriate aldehydes using our original strategy in two steps¹². The condensation and cyclization to originate porphyrinogens follows Lindsey's procedure, but the oxidation of the porphyrinogens is carried out using our recent method of porphyrinogen oxidation with hydrogen peroxide in acetic acid¹⁰ (example 1, 2 and 3, procedure 1).

5,15-diarylporphyrins are synthesized by condensation of the corresponding aryldipyrromethane with trimethylorthoformate as described by Dolphin²³, followed by the oxidation of the porphyrinogen as indicated before with hydrogen peroxide. (example 4, procedure 1).

Relatively to methods of hydroporphyrin synthesis, Whitlock²⁴ described a procedure for chlorins and bacterioclorins that was followed for the synthesis of polar²⁶ and apolar²⁵ chorins.

The reduction of the porphyrins, when Whitlock conditions are used, usually gives macrocycles that are at an higher reduced level than wanted and therefore requires the use of oxidants in a subsequent step to bring them to the chlorin state. This situation originates difficulties in the isolation and affects the overall yield of the process. We improved experimental conditions in order to abolish the need for the oxidation step and to isolate chlorin in better conditions. Those conditions developed by us are described in example 5 procedure 2.A and involve the addition of starting materials, *p-*toluenesulphonylhydrazine and potassium carbonate, at once, in a 6:1 ratio relatively to the porphyrin. After 6-8 hours, the hydrophobic chlorin and some starting porphyrin are recovered from the reaction medium by precipitation with water. The porphyrin and chlorin are easily isolated by chromatographic methods. For the preparation of bacteriochlorin we used a large excess of *p*-toluenesulfonylhydrazine, 40:1 relatively to porphyrin, which is added in two portions as described in example 8 of procedure 2.E. Using this method for reduction, the isolation of the product is achieved by water addition and allows the bacteriochlorin to be directly isolated from the reaction medium in 85% yield. The bacteriochlorin obtained is only contaminated with small amounts of the corresponding chlorin.

Although the process described above represents a significant improvement to the preparation of chlorins and bacteriochlorins, we developed a new process of porphyrin reduction that avoids the use of a base. In this process the diimide is generated by thermal cleavage of the *p*-toluenesulphonylhydrazine at temperatures above 140°C. Using xylene as solvent and a 4:1 ratio of *p*-toluenesulphonylhydrazine relatively to porphyrin, this process gives high yields of chlorins. With the use of a higher ratio of p-toluenesulfonylhydrazine relatively to the porphyrin (40:1) and addition in two portions, this process originated yields of bacteriochlorins of over 85%.
One of the great advantages of this reduction process is the easier isolation of the products by washing the organic phase with water followed by solvent evaporation. (example 5, procedure 2.B)

An extension of this process to generate hydroporphyrin through diimide thermal cleavage makes use of mesitylenesulphonylhydrazine and tetrahydrofuran or methanol as solvent. The details corresponding to the use of this reduction reagent are described in example 5 procedure 2.C for chlorin synthesis and example 8, procedure 2.F for bacteriochlorin synthesis. Only with this last procedure was it possible to perform the reduction of polar porphyrins that are less soluble in xylene. This fact is very important when the products are bacteriochlorins since chlorins are less soluble than the corresponding porphyrins.

An additional novelty in the hydroporphyrin syntheses presented in this invention is the process of chlorin production developed by controlled oxidation of the porphyrinogen instead of reduction of the porphyrin. We observed that porphyrinogens with bulky substituents at *ortho* positions of the *meso*-phenyl groups can suffer controlled oxidation to the chlorin stage alternatively to oxidation to porphyrin level. Example 6, procedure 2.D describes the conditions for the controlled oxidation of a porphyrinogen.

The following scheme illustrates the different synthetic pathways included in this patent for the synthesis of porphyrins, chlorins and bacteriochlorins using the tetrapyrrolic 5,10,15,20-tetrakis (2-bromo-5-hydroxyphenyl) macrocycle as an example.

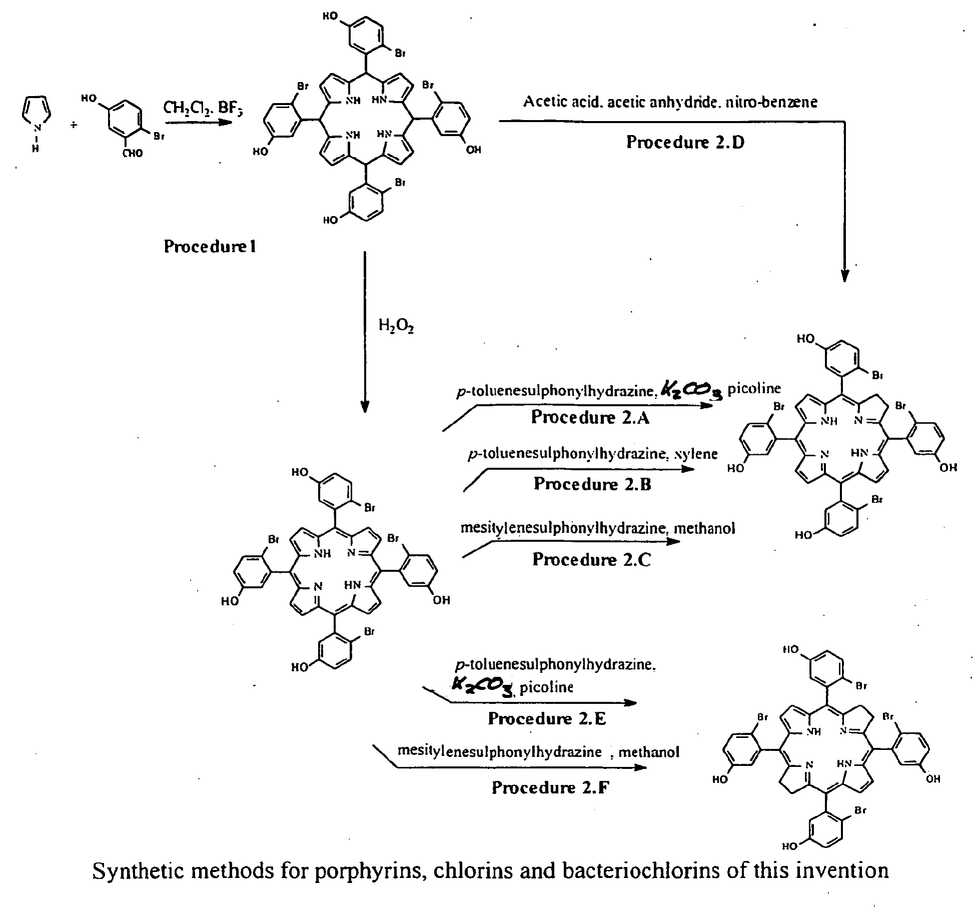

The compounds described in this invention can be used for the preparation of pharmaceutical compositions that can be administered to humans or animals. These compositions can be run via enteric, parenteric or transdermic way, and can also be presented as tablets, pills, capsules, suspensions or solutions (oral and intravenous), dermic ointment or transdermic band-aid, in which the compound can be associated with additives and/or excipients usually employed in the pharmaceutical art. The doses can be comprised between 0.1 and 20 mg/kg body weight.

### Example 1: Synthesis of 5,10,15,20-tetrakis(2-bromo-5-hydroxyphenyl)porphyrin

Procedure 1. 2-Bromo-5-hydroxybenzaldehyde (1.01g, 5.0 mmol) and freshly distilled pyrrole (0.34 mL. 5.0 mmol) were added to 500 ml of degassed and distilled dichloromethane. To this solution 200 µL of a solution of 0.25 ml of boron trifluoroetherate in 1 ml of dichloromethane were added. The reaction was left for 2 hours and then ended by adding 25 µl of triethylamine. This solution was added dropwise to a solution of 100 ml of acetic acid, 5 ml of acetic anhydride and 3 ml of hydrogen peroxide (30%) at 40 °C and left for 1 hour. The solvent was removed by evaporation, the residue dissolved in ethyl acetate, washed with water and dried over sodium sulphate. The filtered solution was concentrated and the residue chromatographed in silica-gel (Kieselgel-60 Merck) with dichloromethane/ethyl acetate (1:1) as eluent giving 270 mg (0.3 mmol) of 5,10,15,20-tetrakis(2-bromo-5-hydroxyphenyl)porphyrin as a purple powder. Recrystalization from dichloromethane-cyclohexane gave purple crystals.

NMR-¹H (CD₃OD) δ-ppm: 7.19-7.30(4H, m, H_{phenyl}), 7.55-7.72(4H, m, H_{phenyl}), 7.83-7.86(4H, m, H_{phenyl}), 8.8(8H, s, H_{pyrrol})

Elementary analysis: calculated for C₄₄H₂₆O₄Br₄N₄.H₂O; N:5.53, C:52.2, H:2,79; found: N:4.71, C:52.5, H:3.13.

UV-Vis absorption (CHCl₃) λₘₐₓ-nm: 415, 511, 542, 587, 642
τ_{T}(N₂) = 33 µs
τ_{T}(N₂) = 540 ns
kq(M⁻¹ s⁻¹) = 1.01x10⁹

### Example 2. Synthesis of 5,10,15,20-tetrakis(2-iodo-5-hydroxyphenyl)porphyrin.

Procedure 1. 2-Iodo-5-hydroxybenzaldehyde (1.23 g, 5.0 mmol) and freshly distilled pyrrole were treated as described in example 1. Chromatography of the reaction product in silica gel (Kieselgel-60 Merck) eluted with dichloromethane/ethyl acetate (1:1) yielded 240 mg (0.2 mmol) of 5,10,15,20-tetrakis(2-bromo-5-hydroxyphenyl)porphyrin as a purple solid. Recrystallization from chloroform/cyclohexane gave purple crystals.

NMR-¹H (CD₃OD) δ-ppm: 7.35(4H, m, H_{phenyl}), 7.64(4H, m, H_{phenyl}), 7.82-7.99(4H, m, H_{phenyl}), 8.91(8H, s, H_{pyrrol})

Elementary analysis: calculated for C₄₄H₂₆O₄I₄N₄.4H₂O; N:4.47, C:42.1, H:2,73; found: N:3.78, C:41.9, H:2.35.

UV-Vis absorption (CHCl₃) λₘₐₓ-nm: 418, 513, 548, 589, 645
τ_{T}(N₂) = 2 µs
τ_{T}(N₂) = 300 ns
kq(M⁻¹ s⁻¹) = 1.60x10⁹

### Example 3. Synthesis of 5,10,15,20-tetrakis(2,4,6-tribromo-5-hydroxyphenyl)porphyrin

Procedure 1. 2,4,6-Tribromo-5-methoxybenzaldehyde (2.24 g, 6.0 mmol) and freshly distilled pyrrole were added to 500 ml of distilled dichloromethane and the solution was purged with N₂. To this solution was added 200 µl of a solution of 0.25 ml of boron trifluoroetherate in 1 ml of dichloromethane. The reaction was left in the dark for 3 hours and terminated by the addition of 25 µl of triethylamine. This solution was concentrated until the volume was 200 ml and a solution of 100 ml of acetic acid, 5 ml of acetic anhydride and 3 ml of hydrogen peroxide (30%) was added drop by drop at 40-50 °C and kept for 1 hour. The solvent was removed and the product dissolved in ethyl acetate. After washing with water the organic layer was dried over anhydrous sodium sulphate, filtered and concentrated. The residue was chromatography on Silica gel (Kieselgel-60 Merck) eluted with dichloromethane/ethyl acetate (8:2) yielding 450 mg (0.3 mmol) of 5,10,15,20-tetrakis(2,4,6-tribromo-5-methoxyphenyl)porphyrin as a purple solid that was recrystallized from chloroform/cyclohexane.

NMR-¹H (CDCl₃) δ-ppm: -2.51 (2H, s, NH) 4.11(12H, s, CH₃), 8.23(4H, s, H_{phenyl}), 8.64(8H, s, H_{pyrrol})

UV-Vis absorption (CH₂Cl₂) λₘₐₓ-nm: 421, 516, 589, 659 200 mg of 5,10,15,20-tetrakis(2,4,6-tribromo-5-methoxyphenyl)porphyrin was added to 3-4 g of pyridine hydrochloride and refluxed for 3 hours. After cooling the product was poured over water and extracted with ethyl acetate. The organic layer was washed first with acidified water and then with water. After evaporation of the solvent the solid was recrystallized with chloroform/methanol yielding 70 mg of 5,10,15,20-tetrakis(2,4,6-tribromo-5-hydroxyphenyl)porphyrin as purple crystals.

UV-Vis absorption (CHCl₃) λₘₐₓ-nm: 416, 514, 550, 589, 644.

### Example 4. Synthesis of 5,15-bis(2-bromo-5-hydroxyphenyl)porphyrin

Procedure 1. The 5,15-bis(2-bromo-5-hydroxyphenyl)porphyrin was prepared following the method described by Dolphin *el al*²³*.* A solution of 2.2 g of trifluoroacetic acid in 25 ml of dichloromethane was slowly added to a solution of 610 mg of 5-(2-bromo-5-acetoxyphenyl)dipyrromethane on 500 ml of dichloromethane and 4 ml of trimethyl orthoformate. After 4 hours 1.05 ml of pyridine was added and the solution keep on the dark for 16 hours. The solution was poured over a solution of 50 ml of acetic acid and 5 ml of acetic anhydride, the dicloromethane was evaporated and 2 ml of hydrogen peroxide and 10 ml of acetic acid were added to the crude material.
The organic layer was washed with water, sodium hydrogen carbonate (5%) and again with water. After drying over anhydrous sodium sulphate the solution was concentrated and the product was chromatographed on Silica gel (Kieselgel-60 Merck) using dichloromethane/hexane/triethylamine (90:9:1) as eluent. The first band collected was 5,15-bis(2-bromo-5-acetoxyphenyl)porphyrin (71 mg), which was recrystallized with dichloromethane/methanol.

NMR-¹H (CDCl₃) δ-ppm: -3.16 (2H, s, NH) 2.44(6H, s, CH₃), 7.05-8.10(6H, s, H_{phenyl}), 8.98(4H, d, H_{pyrrol}, J = 4.6Hz), 9.42 (4H, d, H_{pyrrol}, J = 4.6 Hz), 10.34 (2H, s, Hmeso)

UV-Vis absorption (CH₂Cl₂) λₘₐₓ-nm: 407, 454, 501, 532, 573, 628

The solution of 5,15-bis(2-bromo-5-acetoxyphenyl)porphyrin (71 mg) in 20 ml of ethanol and 90 mg of sodium hydroxide was refluxed for 1 hour. After cooling, neutralizing with acetic acid and evaporating, the crude product was dissolved in 100 ml of dichloromethane and washed with sodium hydrogen carbonate (5%) and water. After drying over anhydrous sodium sulphate the organic layer was evaporated. The product was recrystallized with chloroform/cyclohexane, yielding 47 mg of 5,15-bis(2-bromo-5-hydroxyphenyl)porphyrin.

NMR-¹H (CDCl₃) δ-ppm: 6.85-8.05(6H, m, H_{pheny}), 8.85-8.93(4H, s, H_{pyrrol}), 9.26-9.33(4H, m, H_{pyrrol}), 10.18-10.22(2H, m, Hₘₑₛₒ)

UV-Vis absorption (CH₂Cl₂/Triethylamine) λₘₐₓ-nm: 407, 501, 532, 573, 627

### Example 5. Synthesis of 5,10,15,20-tetrakis(2-bromo-5-hydroxyphenyl)chlorin

Procedure 2.A: 50 mg (0.05 mmol) of 5,10,15,20-tetrakis(2-bromo-5-hydroxyphenyl)porphyrin, 74.4 mg (0.4 mmol) of *p*-toluenesulphonylhydrazine and 54.4 mg (0.4 mmol) of anhydrous potassium carbonate were dissolved in 50 ml of α-picoline saturated with N₂ and heated to reflux. The reaction was monitored by UV-Vis spectroscopy, using aliquots of the reaction mixture diluted with α-picoline, and stopped when the absorption band at 742 nm, typical of the bacteriochlorin was observed. After cooling, 50 ml of water was added, and the reaction product extracted with CHCl₃/CH₃OH (10:1). The organic layer was concentrated and washed with water (20 ml x 3), dried with anhydrous Na₂SO₄ and the solvent evaporated. The reaction product was recrystallized with diethyl ether/n-hexane.

The ¹H-NMR analysis of the reaction product, showed that, it only contained the initial porphyrin and the chlorin in a ratio of 40:60.

The chlorin was purified by chromatography over silica gel using chloroform as eluent; after collection of the first fraction, the eluent was changed to chloroform/ethyl acetate (5:1) to collect the chlorin. This procedure yields 53% of 5,10,15,20-tetrakis(2-bromo-5-hydroxyphenyl)chlorin.

Procedure 2.B: 50 mg (0.05 mmol) of 5,10,15,20-tetrakis(2-bromo-5-hydroxyphenyl)porphyrin and 372 mg (0.2 mmol) of *p*-toluenesulphonylhydrazine were dissolved in 100 ml of xylene saturated with N₂ and heated to reflux. After 4 hours the reaction yielded the chlorin as a green precipitate on the reaction flask. After cooling, the precipitate was filtered (yielding 48 mg) and analysed by ¹H-NMR spectroscopy. The crude material contained 58% of the desired chlorin. The chlorin was purified by column chromatography using silica gel (Kieselgel 60-Merck) and as mobile phase CHCl₃ changing to CHCl₃/ethyl acetate (5:1) after collecting the first fraction.

Procedure 2.C: 0.2 mmol of 5,10,15,20-tetrakis(2-bromo-5-hydroxyphenyl)porphyrin and mesitylenesulphonylhydrazine were dissolved in 100 ml of dried and distilled methanol and refluxed for 6 hours. After cooling, the methanol was evaporated and the reaction product was dissolved in chloroform, washed with water and dried with anhydrous sodium sulphate. The crude residue contained some of the initial porphyrin and the desired chlorin. The chlorin was purified by the method described in procedure 2.A.

### Example 6. Synthesis of 5,10,15,20-tetrakis(2-bromo-5-hydroxyphenyl)chlorin (oxidative route).

Procedure 2.D: The porphyrinogen was prepared as described in example 1. The oxidation was performed by addition of the porphyrinogen solution in dichloromethane to a solution of acetic acid/acetic anhydride/nitrobenzene (100:5.5 ml) at 50-60°C, allowing the dichloromethane immediately. At the end of the dichloromethane addition the solution was heated at 100°C for 8 hours, then 100 ml of water was added and the nitrobenzene removed by steam distillation. Na₂CO₃ was added until complete precipitation. The filtered material was dissolved in ethyl acetate and chromatographed on Silica gel (Kieselgel-60 Merck) using dichloromethane/ethyl acetate (1:1) as eluent to yield 310 mg of a porphyrin/chlorin mixture which was recrystallized in dichloromethane/cyclohexane. The NMR of this mixture shows that it contains 57% of 5,10,15,20-tetrakis(2-bromo-5-hydroxyphenyl)porphyrin and 43% of 5,10,15,20-tetrakis(2-bromo-5-hydroxyphenyl)chlorin. The purification of the chlorin could be carried out according to the procedure described on the procedure 2.A, 2.B and 2.C (example 5). 5,10,15,20-tetrakis(2-bromo-5-hydroxyphenyl)chlorin has the following spectroscopy characteristics.

NMR-¹H (CDCl₃: CD₃OD) δ-ppm: -1.47 (2H, s, NH) 4.13(4H, s, H_{pyrrol}), 7.98-7.32(12H, m, H_{phenyl}), 8.15(2H, s, H_{pyrrol}), 8.31 (2H, d, H_{pyrrol}), 8.51 (2H, s, H_{pyrrol})

UV-vis absorption (CH₃OH) λₘₐₓ-nm: 415, 512, 540, 588, 655

### Example 7. Synthesis of 5,10,15,20-tetrakis(2-iodo-5-hydroxyphenyl)chlorin (oxidative route).

Procedure 2.D: Using an analogous procedure to that described in example 6 a mixture of 60% of chlorin and 40% of porphyrin was obtained.

### Example 8. Synthesis of 5,10,15,20-tetrakis(2-bromo-5-hydroxyphenyl)bacteriochlorin.

Procedure 2.E: 50 mg (0,05 mmol) of 5,10,15,20-tetrakis(2-bromo-5-hydroxyphenyl)porphyrin, 372 mg (2 mmol) of *p*-toluenesulphonylhydrazine and 272 mg (2 mmol) of anhydrous potassium carbonate were dissolved in 50 ml of α-picoline saturated with N₂ and the temperature was increased until reflux. The reaction was followed by UV-Vis spectroscopy using aliquots of the reaction diluted with α-picoline, and following the increase of the absorption band at 742 nm. When the increase of the absorption slows down, 372 mg of *p*-toluenesulphonylhydrazine was added and the reaction conditions maintained until the absorption band at 742 nm raised to the maximum. At this moment the reaction was cooled to room temperature. Then 50 ml of water were added and the reaction product was extracted with chloroform/methanol (10:1). The organic layer was concentrated and washed with water (20 ml x 3), dried over anhydrous sodium sulphate and the solvent was evaporated. The residue was recrystallized with diethyl ether/hexane.

The analysis of the reaction material by UV-Vis spectroscopy showed that the product was only a mixture of 5,10,15,20-tetrakis(2-bromo-5-hydroxyphenyl)chlorin and 5,10,15,20-tetrakis(2-bromo-5-hydroxyphenyl)bacteriochlorin in a ratio of (50:50).

The bacteriochlorin was purified by column chromatography using silica gel (Kieselgel-60 Merck) and CHCl₃/n-hexane as eluent, increasing the polarity gradually to CHCl₃/hexane/ethyl acetate (4:2:1).

UV-Vis absorption (CH₂Cl₂) λₘₐₓ-nm: 361, 374, 514, 738.

Procedure 2.F: The polar bacteriochlorins could be synthetised dissolving the porphyrin in 100 ml of dried and distilled methanol (or THF), with mesitylenesulphonylhydrazine at reflux using a ratio of porphyrin/mesitylenesulphonylhydrazine (1:60), being the mesitylenesulphonylhydrazine added in three times at intervals of 6 hours. The bacteriochlorin was obtained in a very pure state.

### References

1)Bonnett, R. Chem. Soc. Rev. 1995, 24, 19-33.
2)Bonnett, R. Chemical Aspects of Photodynamic Therapy; Gordon and Breach science Publishers: Amsterdam, 2000; Vol. 1.
3)Dougherty, T. J. Photochem. Photobiol. 1993, 58, 895-900.
4)Jori, G.J. Photochem. Photobiol. A: Chem. 1992, 62, 371.
5)MacDonald, I. J.; Dougherty, T. J. J. of Porphyrins and Phthalocyanines 2001, 5, 105-129.
6)Abels, C.; Goezt, A. E. A Clinical Protocol for Photodynamic Therapy; Abels, C.; Goezt, A. E., Ed.; OEMF spa: Milano, 1996, pp 265-281.
7)Reddi, E. Strategies Adopted for Optimizing the Photodynamic Therapy of Tumors; Reddi, E., Ed.; OEMF spa: Milano, 1996, pp 247-264.
8)Redmond, R. W.; Gamlin, J. N. Photochem. Photobiol. 1999, 70, 391-475.
9)Gorman, A. A. The Bimolecular Reactivity of Singlet Molecular Oxygen; Gorman, A. A., Ed.; John Wiley & Sons, Inc.: New York, 1992; Vol. 17, pp 217-274.
10)Johnstone, R. A. W.; Nunes, M. L. P. G.; Pereira, M. M.; Rocha Gonsalves, A. M. d'A.; Serra, A. C. Heterocycles 1996, 43,1423-1437.
11)Rocha Gonsalves, A. M. d'A.; Varejão, J. M. T. B.; Pereira, M. M. J. Heterocyclic Chem. 1991, 28, 635-640.
12)Rocha Gonsalves, A. M. d'A.; Pereira, M. M. J. Heterocyclic Chem. 1985, 22, 931-933.
13)Rocha Gonsalves, A. M. d'A., Synthetic Porphyrins for Haemoprotein Studies. In Chemistry Department; Universidade de Liverpool: Liverpool, 1972.
14)Pineiro, M.; Carvalho, A. L.; Pereira, M. M.; Rocha Gonsalves, A. M. d'A.; Arnaut, L. G.; Formosinho, S. J. Chem. Eur. J. 1998, 4, 2299-2307.
15)Pineiro, M.; Pereira, M. M.; Rocha Gonsalves, A. M. d'A.; Arnaut, L. G.; Formosinho, S. J. J. Photochem. Photobiol. A: Chem 2000, 138, 147-157.
16)Pineiro, M.; Arnaut, L. G.; Formosinho, S. J.; Rocha Gonsalves, A. M. d'A., Polish Journal of Medical Physics and Engineering 2001, 6, 177-184.
17)Pineiro, M.; Rocha Gonsalves, A. M. d'A.; Pereira, M. M.; Formosinho, S. J.; Arnaut, L. G. J. Phys. Chem. 2001, in press.
18)Pineiro, M. Estudo de Modelação de Macrociclos Tetrapirrólicos como Sensibilizadores Fotoquimicos. In Departamento de Química; Universidade de Coimbra: Coimbra, 2001.
19)Azenha, E. G.; Serra, A. C.; Pineiro, M.; Pereira, M. M.; Melo, J. S. d.; Arnaut, L. G.; Formosinho, S. J.; Rocha Gonsalves, A. M. d'A., unpublished results, **2001.**
20)Lock, M. Monatshefte Chem. 1930, 55, 307.
21)Hodgson, H. H.; Beard, H. G. J. Chem. Soc. 1925, 127, 875.
22)Lindsey, J. S.; Schreiman, I. C.; Hsu, H. C.; Kearney, P. C.; Marguerettaz, A. M. J. Org. Chem. 1987, 52, 827.
23)Boyle, R. W.; Bruckner, C.; Posakony, J.; James, B. R.; Dolphin, D. Org. Syntheses, CV 76, 287.
24)Whitlock Jr, H. W.; Hanauer, R.; Oester, M. Y.; Bower, B. K. J. Am. Chem. Soc. 1969, 91, 7485-7489.
25)Senge, M. O.; Kalish, W. W.; Runge, S. Tetrahedron 1998, 54, 3781-3798.
26)US-4,837,221 6/1989 Bonnett, R et al; US-4,992,257 2/1991 Bonnett, R et al*;* US-5,162.519 11/1992 Bonnett R. et al

## Claims

1. Macrocyclic tetrapyrrolic compounds of the family of porphyrins (structure I), chlorins (structure II) and bacteriochlorins (structure III) **characterized by** substitution in positions 5, 10, 15 and 20 of the macrocycle with two or more phenyl groups with bromine in the *ortho* and/or para position and a hydroxyl group in the *meta* position of the phenyl ring; and wherein the positions 5, 10, 15, 20 which are not substituted by the phenyl group are substituted by hydrogen or halogen atoms; and wherein positions 2, 3, 7, 8, 12, 13, 17 and/or 18 of the macrocycle are substituted with hydrogen or halogen atoms or alkyl, vinyl, carboxy, carboxyalkyl, alkoxycarbonyl, alkoxycarbonylalkyl, alkoxycarbonylaryl, acetoxy or hydroxyalkyl groups.

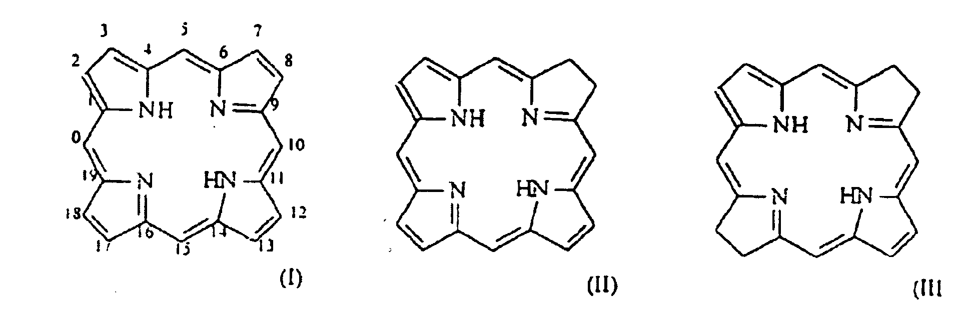

2. Compound according to claim 1, **characterized in that** the compound is 5, 10, 15,20-tetrakis(2-bromo-5-hydroxyphenyl)porphyrin.

3. Compound according to claim 1, **characterized in that** the compound is 5, 10, 15,20-tetrakis(2,4,6-tribromo-5-hydroxyphenyl)porphyrin.

4. Compound according to claim 1, **characterized in that** the compound is 5,15-bis(2-bromo-5-hydroxyphenyl)porphyrin.

5. Compound according to claim 1, **characterized in that** the compound is 5, 10, 15,20-tetrakis(2-bromo-5-hydroxyphenyl)chlorin.

6. Compound according to claim 1, **characterized in that** the compound is 5, 10, 15,20-tetrakis(2-bromo-5-hydroxyphenyl)bacteriochlorin.

7. Compound for use in the photodynamic therapy, **characterized in that** the compound is 5,10,15,20-tetrakis(2-iodo-5-hydroxyphenyl)porphyrin or 5,10,15,20-tetrakis(2-iodo-5-hydroxyphenyl)chlorin.

8. The processes for the synthesis of porphyrins according to any of the claims 1, 2, 3 or 4, **characterized by** the corresponding porphyrinogen being oxidized with hydrogen peroxide.

9. The processes for the synthesis of chlorins and bacteriochlorins according to any of the claims 1,3,6 and 7, **characterized by** the reduction of the corresponding porphyrin with diimide generated by thermal decomposition of *p-*toluenesulphonylhydrazine or mesitylenesulphonylhydrazine.

10. The process for the synthesis of chlorins according to any of the claims 1,3 and 6, **characterized by** the controlled oxidation of the corresponding porphyrinogen in acetic acid in the presence of nitrobenzene.

11. The compounds according to any of the claims 1,2,3,4,5,6 or 7 for use as a medicament.

12. The medicament according to claim 11, **characterized in that** the medicament further comprises an appropriate additive.

13. Medicament comprising the compounds according to any of the claims 1 to 7 for use in the photodynamic therapy and photodiagnosis.

## Patentansprüche

1. Makrozyklische Tetrapyrrol-Verbindungen aus der Familie der Porphyrine (Struktur I), Chlorine (Struktur II) und Bakteriochlorine (Struktur III), **gekennzeichnet durch** Substitution in den Positionen 5, 10, 15 und 20 des Makrozyklus mit zwei oder mehreren Phenylgruppen mit Brom in der *ortho*- und/oder *para*-Position und einer Hydroxylgruppe in der *meta*-position des Phenylrings; und wobei die Positionen 5, 10, 15, 20, welche nicht mit der Phenylgruppe substituiert sind, mit Wasserstoff- oder Halogenatomen substituiert sind; und wobei die Positionen 2, 3, 7, 8, 12, 13, 17 und/oder 18 des Makrozyklus mit Wasserstoff- oder Halogenatomen oder Alkyl-, Vinyl-, Carboxy-, Carboxyalkyl-, Alkoxycarbonyl-, Alkoxycarbonylalkyl-, Alkoxycarbonylaryl-, Acetoxy- oder Hydroxyalkylgruppen substituiert sind.

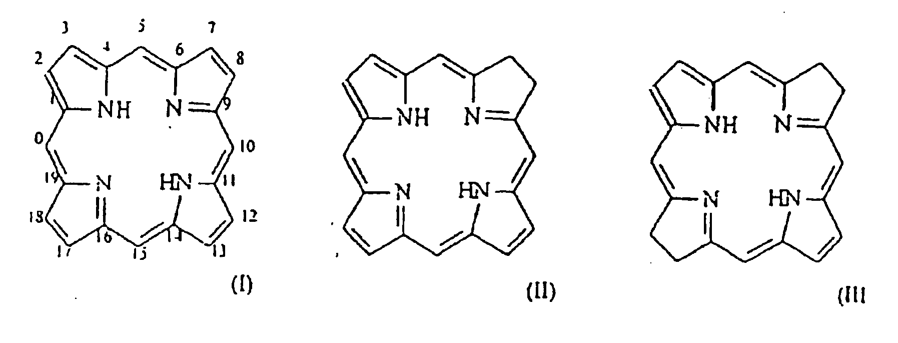

2. Verbindung gemäß Anspruch 1, **dadurch gekennzeichnet, dass** die Verbindung 5,10,15,20-Tetrakis(2-brom-5-hydroxyphenyl)porphyrin darstellt.

3. Verbindung gemäß Anspruch 1, **dadurch gekennzeichnet, dass** die Verbindung 5,10,15,20-Tetrakis(2,4,6-tribrom-5-hydroxyphenyl)porphyrin darstellt.

4. Verbindung gemäß Anspruch 1, **dadurch gekennzeichnet, dass** die Verbindung 5,15-Bis(2-brom-5-hydroxyphenyl)porphyrin darstellt.

5. Verbindung gemäß Anspruch 1, **dadurch gekennzeichnet, dass** die Verbindung 5,10,15,20-Tetrakis(2-brom-5-hydroxyphenyl)chlorin darstellt.

6. Verbindung gemäß Anspruch 1, **dadurch gekennzeichnet, dass** die Verbindung 5,10,15,20-Tetrakis(2-brom-5-hydroxyphenyl)bakteriochlorin darstellt.

7. Verbindung zur Verwendung in der photodynamischen Therapie, **dadurch gekennzeichnet, dass** die Verbindung 5,10,15,20-Tetrakis(2-iod-5-hydroxyphenyl)porphyrin oder 5,10,15,20-Tetrakis(2-iod-5-hydroxyphenyl)chlorin darstellt.

8. Die Verfahren zur Synthese der Porphyrine gemäß irgendeinem der Ansprüche 1, 2, 3 oder 4, **dadurch gekennzeichnet, dass** der entsprechende Porphyrinvorläufer mit Wasserstoffperoxid oxidiert wird.

9. Die Verfahren zur Synthese von Chlorinen und Bakteriochlorinen gemäß irgendeinem der Ansprüche 1, 3, 6 und 7, **gekennzeichnet durch** die Reduktion des korrespondierenden Porphyrins mit einem Diimid, welches **durch** die thermische Zersetzung von *p*-Toluolsulphonylhydrazin oder Mesitylensulphonylhydrazin erhalten wurde.

10. Das Verfahren zur Synthese von Chlorinen gemäß irgendeinem der Ansprüche 1, 3 und 6, **gekennzeichnet durch** die kontrollierte Oxidation des entsprechenden Porphyrinvorläufers in Essigsäure in Gegenwart von Nitrobenzol.

11. Die Verbindungen gemäß irgendeinem der Ansprüche 1, 2, 3, 4, 5, 6 oder 7 zur Verwendung als Medikament.

12. Das Medikament gemäß Anspruch 11, **dadurch gekennzeichnet, dass** das Medikament weiterhin ein geeignetes Additiv umfasst.

13. Medikament umfassend die Verbindungen gemäß irgendeinem der Ansprüche 1 bis 7 zur Verwendung in der photodynamischen Therapie oder Photodiagnose.

## Revendications

1. Composés tétrapyrroliques macrocycliques de la famille des porphyrines (structure I), des chlorines (structure II) et des bactériochlorines (structure III), **caractérisés par** la substitution en positions 5, 10, 15 et 20 du macrocycle avec deux ou plus de deux groupes phényles ayant un brome en position *ortho* et/ou *para* et un groupe hydroxyle en position *méta* du cycle phényle ; et dans lesquels les positions 5, 10, 15, 20 qui ne sont pas substituées par le groupe phényle sont substituées avec des atomes d'hydrogène ou d'halogène ; et dans lesquels les positions 2, 3, 7, 8, 12, 13, 17 et/ou 18 du macrocycle sont substituées avec des atomes d'hydrogène ou d'halogène ou des groupes alkyle, vinyle, carboxy, carboxyalkyle, alcoxycarbonyle, alcoxycarbonylalkyle, alcoxycarbonylaryle, acétoxy ou hydroxyalkyle.

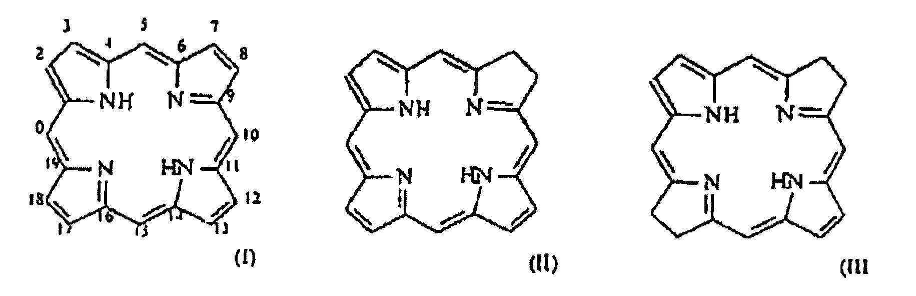

2. Composé selon la revendication 1, **caractérisé en ce que** le composé est la 5,10,15,20-tétrakis(2-bromo-5-hydroxyphényl)porphyrine.

3. Composé selon la revendication 1, **caractérisé en ce que** le composé est la 5,10,15,20-tétrakis(2,4,6-tribromo-5-hydroxyphényl)porphyrine.

4. Composé selon la revendication 1, **caractérisé en ce que** le composé est la 5,15-bis(2-bromo-5-hydroxyphényl)porphyrine.

5. Composé selon la revendication 1, **caractérisé en ce que** le composé est la 5,10,15,20-tétrakis(2-bromo-5-hydroxyphényl)chlorine.

6. Composé selon la revendication 1, **caractérisé en ce que** le composé est la 5,10,15,20-tétrakis(2-bromo-5-hydroxyphényl)bactériochlorine.

7. Composé utilisable en thérapie photodynamique, **caractérisé en ce que** le composé est la 5,10,15,20-tétrakis(2-iodo-5-hydroxyphényl)porphyrine ou la 5,10,15,20-tétrakis(2-iodo-5-hydroxyphényl)chlorine.

8. Procédés de synthèse de porphyrines selon l'une quelconque des revendications 1, 2, 3 ou 4, **caractérisés en ce que** le porphyrinogène correspondant est oxydé avec du peroxyde d'hydrogène.

9. Procédés de synthèse de chlorines et de bactériochlorines selon l'une quelconque des revendications 1, 3, 6 et 7, **caractérisés par** la réduction de la porphyrine correspondante avec un diimide généré par la décomposition thermique de la *p*-toluènesulfonylhydrazine ou de la mésitylènesulfonylhydrazine.

10. Procédé de synthèse de chlorines selon l'une quelconque des revendications 1, 3 et 6, **caractérisé par** l'oxydation contrôlée du porphyrinogène correspondant dans de l'acide acétique en présence de nitrobenzène.

11. Composés selon l'une quelconque des revendications 1, 2, 3, 4, 5, 6 ou 7, pour un usage en tant que médicament.

12. Médicament selon la revendication 11,
**caractérisé en ce que** le médicament comprend en outre un additif approprié.

13. Médicament comprenant les composés selon l'une quelconque des revendications 1 à 7, pour un usage en thérapie photodynamique et en photodiagnostic.
